(19) Europäisches Patentamt
European Patent Office
Office européen des brevets

(11) Publication number: 0 267 627 B1

(12) EUROPEAN PATENT SPECIFICATION

(45) Date of publication of patent specification: 06.03.91

(51) Int. Cl.$^5$: C07C 43/12, C07C 43/313, C07C 41/06

(21) Application number: 87116813.4

(22) Date of filing: 13.11.87

# (54) Process for the preparation of halogenated polyethers.

(30) Priority: 14.11.86 IT 2234786

(43) Date of publication of application:
18.05.88 Bulletin 88/20

(45) Publication of the grant of the patent:
06.03.91 Bulletin 91/10

(84) Designated Contracting States:
DE FR GB NL

(56) References cited:
EP-A- 0 201 871
DD-A- 243 276
GB-A- 2 148 286
US-A- 3 962 348
US-A- 4 500 739

(73) Proprietor: AUSIMONT S.r.l.
31, Foro Buonaparte
I-20100 Milano(IT)

(72) Inventor: Marraccini, Antonio, Dr.
4, corso Riviera
I-28040 Dormelletto (Novara)(IT)
Inventor: Guastalla, Giovanni, Dr.
63/A, via Fara
I-28100 Novara(IT)
Inventor: Malacrida, Alessandro, Dr.
49, via Lambro
I-20050 Sovico (Milano)(IT)
Inventor: Guglielmo, Giorgio, Dr.
89, via A. Gramsci
I-30035 Mirano (Venezia)(IT)

(74) Representative: Barz, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
W-8000 München 40(DE)

EP 0 267 627 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention concerns a process for the preparation of halogenated polyethers.

More particularly the polyethers that may be prepared by means of the process of the present invention are of the general formula:

$$R_FO-CFX - CF_2 - OR'_F \qquad (I) \quad \text{and}$$

$$R_FO-\underset{\displaystyle CF_2X}{\underset{|}{C}}F-OR'_F \qquad (II)$$

wherein X is Cl or F, $R_F$ F and $R'_F$, either equal to or different from each other, are selected from fluorinated alkyl radicals having from 1 to 20 carbon atoms, cycloalkyl, heterocyclic, alkylcycloalkyl or cycloalkylalkyl radicals having 5 to 20 carbon atoms, said radicals optionally also containing other halogen atoms different from fluorine, and optionally ether oxygen atoms.

From the literature there are known processes for the preparation of the above mentioned polyethers by means of fluorination of the hydrogenated polymers and by the subsequent breaking up of the polymeric chain.

For instance, US-A-4 523 039 describes a process comprising the synthesis of the fluorinated polyethers, by direct fluorination with gaseous $F_2$ (of the corresponding hydrogenated polyethers) and by the subsequent fragmentation of the polymeric chain thus formed in order to produce perfluoropolyethers of a low molecular weight.

Said process has the drawback of requiring very long times for obtaining the desired product, that is, in the order of various days, in order that the fluorination be completed.

Another known process for obtaining the indicated perfluoropolyethers is the reaction of fluoroolefines with polyols, and the subsequent electrofluorination (see US-A-3 962 348). Also in this case the process requires the use of HF which, as is well known, involves quite a number of technological and security problems.

There has now been found a very simple method which, in addition, does not show the previously above mentioned disadvantages, and is suited for preparing the products of the invention.

Object of the present invention is to prepare compounds particularly suitable for the testing in electronics with a simpler process than those known in the art. See for this purpose the class of compounds disclosed in EP-A-203 348.

It is well known that the compounds suitable for this application have to have a large range between the pour point and the boiling temperature. In any case it is preferable that the pour point be as low as possible.

We have unexpectedly found that the perfluorinated compounds described in the formulae I and II have the properties indicated above. By using the process of the invention, Applicant unexpectedly has found that it is possible to prepare compounds of formulae I and II with a good yield, and above all without other reaction products which would be very difficult to be separated from the product of the invention.

Thus, object of the present invention is to provide a process for obtaining halogenated polyethers of the formula:

$$R_FO-CFX - CF_2 - OR'_F \quad \text{and} \quad R_FO-\underset{\displaystyle CF_2X}{\underset{|}{C}}F-OR'_F .$$

said process comprising reacting a fluorooxy compound or a chlorooxy compound of the formula: $R'_FOX$ with a fluorovinylether having formula: $R_FO-CF=CF_2$, wherein X, $R_F$ and $R'_F$ have the above indicated meanings, said reactants being used in stoichiometric quantities or, optionally, using an excess in perfluorovinylether.

The perfluorovinylether is in a liquid phase, optionally in the presence of an inert solvent, for instance chlorofluorohydrocarbons or perfluorohydrocarbons such as for example Algofrens® 114 and 115.

The reaction temperature may vary within a range of from -30 °C to -140 °C, preferably -50 °C to -100 °C.

The reaction is conducted by keeping the temperature within the indicated range, since it has been observed that thereby the occurrence of decomposition reactions of the hypofluorite or of other secondary reactions can be avoided.

The fluorooxy (or chlorooxy) compound is fed continuously in a gaseous phase, preferably diluted with an inert gas, such as for instance nitrogen, into a reactor containing the liquid perfluorovinylether.

The fluorooxy or the chlorooxy compound may also be used in a liquid phase, preferably in the presence of an inert diluent, such as for example fluorochlorohydrocarbons.

Illustrating examples of fluorooxy and chlorooxy compounds to be used as starting products in the process of the invention, are: fluorooxytrifluoromethane; chlorooxytrifluoromethane; fluorooxypentafluoroethane, chlorooxypentafluoroethane; fluorooxy-2-chloro-tetrafluoroethane; fluorooxy-2,2-dichlorotrifluoroethane; fluorooxy-2,2,2-trichloro-difluoroethane; fluorooxy-2-bromo-tetrafluoroethane; fluorooxy-heptafluoropropane; fluorooxy-perfluoro-n-propoxy-hexafluoropropane; fluorooxy-2-perfluoroethoxy-tetrafluoroethane; fluorooxy-2-perfluoromethoxy-tetrafluoroethane and their mixtures; fluorooxy-2-trifluoromethoxy-hexafluoropropane; fluorooxy-2-tetrafluoroethoxy-hexafluoropropane.

Illustrating examples of perfluorovinylethers to be used as starting products according to this invention include: perfluoromethyl-vinylether; perfluoroethylvinylether, perfluoropropylvinylether; perfluoro-n-butylvinylether; perfluoroisobutylvinylether; perfluorooctylvinylether; 4-iodoperfluorobutylvinylether; β-iodoperfluoroethylvinylether; β-chloroperfluoroethylvinylether; γ-chloroperfluoropropylvinylether; perfluoro-3,6-dimethyl-1,4-dioxanyl-2-vinylether; perfluoro-2-n-propoxy-n-propylvinylether; perfluoro-2-methoxy-n-propylvinylether; perfluoro-2-ethoxy-n-propylvinylether.

The fluorooxy compounds are products well known from literature and more particularly they may be obtained according to a continuous process described in EP-A-194862.

The chlorooxy compounds may in their turn be obtained according to any of the processes known to the art and more particularly may be obtained by the continuous process described in EP-A-259818.

The perfluoroalkylvinylethers are well known products and may be prepared by dehalogenation of the products described in EP-A-201871.

The perfluorinated polyethers of this invention are compounds well known for their exceptional thermal stability, their thermo-oxidative stability and stability to chemical agents, as well as for their non-inflammability property, and offer performances in highly diversified fields and under extremely severe operational conditions.

The perfluoropolyethers known to the art, in general consist in mixtures of products from which it is difficult to obtain the single compounds. In this connection see GB-A-1 226 566. The perfluoropolyethers of the present invention, on the contrary, are obtainable as compounds, in general as isomeric mixtures having boiling points comprised within a very narrow range of temperatures.

The perfluoropolyethers of the invention are particularly useful, as stated above, as fluids for testing in fields of electronics, for instance for leak-testing, thermal-shock testing, in hot-spot locations, dew-point determination.

The polyethers containing bromine and/or iodine polyethers are used as intermediates for the obtention of functionalized derivatives.

EXAMPLE 1

Into a reactor loaded with 75 ml of 1,2-dichlorotetrafluoroethane and 25 g of perfluoropropylvinylether, and cooled down to a temperature of -75 °C, were continuously fed in 2,5 Nl/h of $CF_3OF$ diluted with 1.5 Nl/h of $N_2$.

In the course of the reaction there were added further 148 g of perfluoropropylvinylether, whi le maintaining the reaction temperature constant at -75 °C.

After 5 hours and 30 minutes, the feeding in of the reactants was stopped and the condensate was subjected to distillation. Thereby were obtained 126 g of a 99 % by weight fraction gathered in the temperature range of 61 - 65 °C and having a pour point of about -138 °C.

The yield was 60 % in moles, calculated on the $CF_3OF$. The product was identified by means of mass-spectrometry, IR spectrophotometry, $^{19}$F-NMR and was found to consist of a mixture of:

$$CF_3CF_2CF_2-O-CF_2CF_2-OCF_3 \quad \text{and}$$
$$CF_3CF_2CF_2-O-\underset{\displaystyle CF_3}{\underset{|}{C}}F-O-CF_3$$

in a ratio of about 26 to 74 between the linear and the branched isomer determined by gas-chromatography.

## Claims

1. A process for the preparation of halogenated polyethers of the general formula:

$$R_FO-CFX - CF_2 - OR'_F \quad (I) \quad \text{and} \quad R_FO - \underset{\displaystyle CF_2X}{\underset{|}{C}}F - OR'_F \quad (II)$$

wherein X is Cl or F, $R_F$ and $R'_F$, either equal to or different from each other, are selected from fluorinated alkyl radicals having 1 to 20 carbon atoms, cycloalkyl, heterocyclic, alkylcycloalkyl or cycloalkylalkyl radicals having 5 to 20 carbon atoms, said radicals optionally containing other halogen atoms different from fluorine and/or ether oxygen atoms, which process comprises reacting a fluorooxy or a chlorooxy compound of the formula: $R'_FOX$ with a fluorovinylether $R_F$-O-CF=$CF_2$, wherein $R_F$ and $R'_F$ have the above indicated meaning, at a temperature of from -50° C to -140° C.

2. A process according to claim 1, characterized in that the temperature is from -50° C to -100° C.

3. A process according to claim 1 or 2, characterized in that the reaction is conducted in a liquid phase consisting of fluorovinylether optionally in admixture with an inert solvent.

4. A process according to claim 3, characterized in that the reaction solvent is a perfluorohydrocarbon or a chlorofluorohydrocarbon.

5. A process according to claim 3 or 4, characterized in that the fluorooxy or chlorooxy compound is continuously fed into the liquid reaction phase in a gaseous form, preferably diluted with an inert gas.

## Revendications

1. Un procédé pour la préparation de polyéthers halogénés de formules générales:

$$R_FO-CFX - CF_2 - OR'_F \quad (I) \quad \text{et} \quad R_FO - \underset{\displaystyle CF_2X}{\underset{|}{C}}F - OR'_F \quad (II)$$

dans lequel X est un chlore ou un fluor, $R_F$ et $R'_F$, identiques ou différents l'un de l'autre, sont choisis parmi les radicaux alkyle fluorés ayant de 1 à 20 atomes de carbone, cycloalkyle, hétérocyclique, et parmi les radicaux alkylcycloalkyle ou cycloalkylalkyle ayant de 5 à 20 atomes de carbone, lesquels radicaux pouvant également contenir d'autres atomes d'halogène différents du fluor et/ou des atomes d'oxygène d'un éther, ledit procédé comprenant la réaction d'un composé fluorooxy ou chlorooxy de formule $R'_FOX$ avec un fluorovinyléther de formule $R_F$-O-CF=$CF_2$, dans lequel X, $R_F$ et $R'_F$ ont les significations indiquées ci-dessus, le fluorovinyléther étant en phase liquide, à une température comprise entre -30° C et -140° C.

2. Un procédé selon la revendication 1, caractérisé en ce que la température est comprise entre -50 ° C et -100 ° C.

3. Un procédé selon la revendication 1 ou 2, caractérisé en ce que la réaction est effectuée dans une phase liquide formée du fluorovinyléther, éventuellement en mélange avec un solvant inerte.

4. Un procédé selon la revendication 3, caractérisé en ce que le solvant de réaction est un hydrocarbure perfluoré ou un hydrocarbure chlorofluoré.

5. Un procédé selon la revendication 3 ou 4, caractérisé en ce que le composé fluorooxy ou chlorooxy est introduit en continu dans le milieu réactionnel liquide sous forme gazeuse, de préférence dilué avec un gaz inerte.

## Ansprüche

1. Verfahren zur Herstellung von halogenierten Polyethern der allgemeinen Formel:

$$R_FO-CFX-CF_2-OR'_F \quad (I) \quad \text{und} \quad R_FO-\underset{\displaystyle CF_2X}{\underset{|}{CF}}-OR'_F \quad (II)$$

worin X Cl oder F ist, $R_F$ und $R'_F$, gleich oder verschieden voneinander, aus fluorierten Alkylresten mit 1 bis 20 Kohlenstoffatomen, Cycloalkyl-, heterocyclischen, Alkylcycloalkyl- oder Cycloalkylalkyl-Resten mit 5 bis 20 Kohlenstoffatomen ausgewählt sind, wobei die Reste gegebenenfalls andere, von Fluor verschiedene Halogenatome und/oder Ether-Sauerstoffatome enthalten, welches Verfahren die Umsetzung einer Fluoroxv- oder Chloroxyverbindung der Formel $R'_FOX$ mit einem Fluorvinylether der Formel $R_F$-O-CF = $CF_2$, worin X, $R_F$ und $R'_F$ die obige Bedeutung haben und der Fluorvinylether sich in flussiger Phase befindet, bei einer Temperatur von -30 ° C bis -140 ° C umfaßt.

2. Verfahren nach Anspruch 1, dadurch gekennzeichnet, daß die Temperatur von -50 ° C bis -100 ° C beträgt.

3. Verfahren nach Anspruch 1 oder 2, dadurch gekennzeichnet, daß die Reaktion in einer flüssigen Phase, die aus Fluorvinylether, gegebenenfalls in Mischung mit einem inerten Lösungsmittel, besteht, durchgeführt wird.

4. Verfahren nach Anspruch 3, dadurch gekennzeichnet, daß das Reaktionslösungsmittel ein Perfluorkohlenwasserstoff oder ein Chlorfluorkohlenwasserstoff ist.

5. Verfahren nach Anspruch 3 oder 4, dadurch gekennzeichnet, daß die Fluoroxy- oder Chloroxyverbindung der flüssigen Reaktionsphase kontinuierlich in gasförmigem Zustand, vorzugsweise mit einem Inertgas verdünnt, zugeführt wird.